# EUROPÄISCHE PATENTSCHRIFT

(11) **EP 3 256 567 B1**
(45) Veröffentlichungstag und Bekanntmachung des Hinweises auf die Patenterteilung: **17.04.2019**
(21) Anmeldenummer: 16703117.8
(22) Anmeldetag: 04.02.2016
(51) Int. Cl.: C12N 1/18, C12N 15/01, C12G 1/022, C12R 1/865

(54) **VERFAHREN ZUR ERZEUGUNG VON HEFE-MUTANTEN UND DEREN VERWENDUNG**
METHOD FOR GENERATING YEAST MUTANTS AND USE THEREOF
PROCÉDÉ DE PRODUCTION DE MUTANTS DE LEVURE ET LEUR UTILISATION

(30) Priorität: 10.02.2015 EP 15154520
(43) Veröffentlichungstag der Anmeldung: 20.12.2017
(73) Patentinhaber: Erbslöh Geisenheim GmbH, 65366 Geisenheim (DE); BRAIN AG, 64673 Zwingenberg (DE)
(72) Erfinder: FRÖHLICH, Jürgen, 55130 Mainz (DE); ECK, Jürgen, 64625 Bensheim-Auerbach (DE); MEURER, Guido, 64342 Seeheim-Jugenheim (DE); NAUMER, Christian, 68161 Mannheim (DE); BÜSCHER, Jörg, 79194 Gundelfingen (DE); MAMPEL, Jörg, 64625 Bensheim (DE)
(74) Vertreter: WSL Patentanwälte Partnerschaft mbB
(86) Internationale Anmeldenummer: PCT/EP2016/052437
(87) Internationale Veröffentlichungsnummer: WO 2016/128296

(56) Entgegenhaltungen:
- EP-A1- 2 634 247
- WO-A1-2011/159894
- INMACULADA BLAZQUEZ ROJAS ET AL: "Influence of choice of yeasts on volatile fermentation-derived compounds, colour and phenolics composition in Cabernet Sauvignon wine", WORLD JOURNAL OF MICROBIOLOGY AND BIOTECHNOLOGY, KLUWER ACADEMIC PUBLISHERS, DO, Bd. 28, Nr. 12, 10. August 2012 (2012-08-10), Seiten 3311-3321, XP035132731, ISSN: 1573-0972, DOI: 10.1007/S11274-012-1142-Y
- V. TILLOY ET AL: "Reduction of Ethanol Yield and Improvement of Glycerol Formation by Adaptive Evolution of the Wine Yeast Saccharomyces cerevisiae under Hyperosmotic Conditions", APPLIED AND ENVIRONMENTAL MICROBIOLOGY, Bd. 80, Nr. 8, 15. April 2014 (2014-04-15) , Seiten 2623-2632, XP055263191, US ISSN: 0099-2240, DOI: 10.1128/AEM.03710-13
- RAJNI KUMARI ET AL: "Improvement of multiple stress tolerance in yeast strain by sequential mutagenesis for enhanced bioethanol production", JOURNAL OF BIOSCIENCE AND BIOENGINEERING, Bd. 114, Nr. 6, 1. Dezember 2012 (2012-12-01), Seiten 622-629, XP055203038, ISSN: 1389-1723, DOI: 10.1016/j.jbiosc.2012.07.007
- WILLS C ET AL: "An efficient selection producing structural gene mutants of yeast alcohol dehydrogenase resistant to pyrazole.", GENETICS AUG 1988, Bd. 119, Nr. 4, August 1988 (1988-08), Seiten 791-795, XP002742423, ISSN: 0016-6731
- SCANES KT ET AL: "Glycerol production by the yeast Saccharomyces cerevisiae and its relevance to wine: a review", J AFR J ENOL VITIC , Bd. 19, Nr. 1 1. Juli 1998 (1998-07-01), Seiten 17-24, XP002742424, Gefunden im Internet: URL:http://www.genetics.org/content/119/4/ 791.full.pdf [gefunden am 2015-07-17]

## Beschreibung

Die vorliegende Erfindung betrifft ein Verfahren zur Erzeugung von Hefe-Mutanten, bei dem man wenigstens einen Hefe-Stamm in einem ersten Mutageneseschritt mit einem ersten Mutagen in Kontakt bringt und in einem zweiten Mutageneseschritt mit einem zweiten Mutagen in Kontakt bringt, sowie Hefen, die durch dieses Verfahren erzeugt wurden, und eine Verwendung derartiger Hefen.

In vielen Weinanbauregionen haben innerhalb der vergangenen Jahre die Temperaturen, während des Zeitraums der Reifung von Weintrauben stark zugenommen, wodurch sich der Zuckergehalt der so gereiften Trauben ebenfalls erhöht hat. Bei der für die Weinerzeugung verwendeten ethanolischen Fermentation, wird dieser Zucker, d. h. insbesondere Glucose, unter Einsatz von Hefe-Stämme zu Ethanol als dem vorherrschenden Fermentationsprodukt umgesetzt. Durch den erhöhten Glucosegehalt in den Trauben in den vergangenen Jahren ist daher bei der ethanolischen Fermentation bei der Erzeugung von Wein das Problem aufgetreten, dass Weine erzeugt wurden, bei denen ein erhöhter Alkoholgehalt vorhanden war. Ein derart erhöhter Alkoholgehalt ist allerdings seitens der Verbraucher unerwünscht, da diese auch in unterschiedlichen Jahrgängen möglichst gleichbleibende Alkoholgehalte einzelner Weine bevorzugen. Zudem hat die Nachfrage nach Weinen mit geringerem Alkoholgehalt in letzter Zeit zugenommen. Weiter stellt Ethanol in Weinen einen Bestandteil dar, der zwar einerseits notwendig und erwünscht ist, der jedoch bei höherem Gehalt dazu führen kann, dass die geschmacklichen Qualitäten des Weines leiden. Positiv auf die Geschmackseigenschaften eines Weines wirken sich hohe Glycerinkonzentrationen, z.B. über 10 g/l, aus.

Ein Ansatz, um diese Eigenschaften bereitzustellen, besteht darin, bekannte Hefen zu verwenden, die bei derselben Menge an Zucker im Most weniger Ethanol produzieren. Zwar sind einige derartige Hefen bekannt, es ist jedoch, insbesondere für die Erzeugung hochqualitativer Weine wünschenswert, eine Auswahl unterschiedlicher Hefen zur Verfügung zu haben, die anstelle des Ethanols unterschiedliche weitere, sekundäre Substanzen produzieren, die wesentlich die Geschmackseigenschaften eines Weines beeinflussen können.

Ansätze, derartige Hefen zu erzeugen, umfassen insbesondere eine gezielte genetische Modifikation sowie herkömmliche Züchtung und an die herkömmliche Züchtung angelehnte Selektionsverfahren. Ein Beispiel für derartige Verfahren zu Erzeugung von Hefen mit gewünschten Eigenschaften ist in WO 2011/080411 offenbart. Ein Nachteil spezifisch genetisch modifizierter Organismen besteht darin, dass Verbraucher diesen Organismen gegenüber sehr skeptisch sind. Züchtungsverfahren und an die herkömmliche Züchtung angelehnte Verfahren sowie reine Selektionsverfahren sind zwar ebenfalls praktikabel, jedoch äußerst langwierige Verfahren und dadurch sehr aufwändig. Alternativ werden auch Wildhefen isoliert und auf die gewünschten Eigenschaften untersucht. Eine derartige Isolierung ist in EP 2 634 247 B1 beschrieben. Auch dieses Verfahren ist sehr aufwändig und der Erfolg nur ungewiss.

V. Tilloy, et al. beschreiben in "Reduction of Ethanol Yield and Improvement of Glycerol Formation by Adaptive Evolution of the Wine Yeast Saccharomyces cerevisiae under Hyperosmotic Conditions" (Applied and Environmental Microbiology, US, Band 80, Nr. 8, S. 2623 - 2632) die adaptive Evolution des Saccharomyces cerevisiae-Stamms LALVIN EC1118 über 200 Generationen in einer Lösung von KCL und die anschließende Hybridisierung der auf diese Weise erhaltenen Hefen untereinander.

In WO 2011/159894 A1 wird ein Verfahren beschrieben, dass auf die Erzeugung von Hefe-Mutanten zielt, bei denen die Sensitivität gegenüber Butanol so verringert ist, dass man bei der Butanol-Produktion höhere Butanol-Ausbeuten erzielen kann. Hierfür erfolgt über zehn Runden alternierend eine Exponierung gegenüber den beiden nukleotid-alkylierenden Agenzien EMS und NTG (syn. MNNG) und zwischendurch die Selektion mit einem Hefe-Medium, das 1% Isobutanol enthält.

Rajni Kumari, et al. beschreiben in "Improvement of multiple stress tolerance in yeast strain by sequential mutagenesis for enhanced bioethanol production" (Journal of Bioscience and Bioengineering, Band 114, Nr. 6, S. 622 - 629) ein Verfahren, dass auf die Erzeugung von multistresstoleranten Hefestämmen zielt, die höhere Ausbeuten bei der Bio-Ethanol-Erzeugung liefern. Die hierfür eingesetzten Selektionsfaktoren Ethanol, hohe Temperaturen und toxische Substanzen, wie z.B. Vanillin, Furfural und Essigsäure dienen der Erzeugung von Hefe-Mutanten, die gegenüber dem jeweiligen Stressfaktor eine höhere Toleranz aufweisen.

Inmaculada Blazquez Rojas, et al. beschreiben in "Influence of choice of yeasts on volatile fermentation-derived compounds, colour and phenolics composition in Cabernet Sauvignon wine" (World Journal of Microbiology and Biotechnology, Band 28, Nr. 12, S. 3311 - 3321) die Verwendung verschiedener Saccharomyces-Stämme für die Herstellung von Wein aus Traubenmost, und unter Verwendung dieser Hefen wird nach 14-32 Tagen Gärung Wein mit Ethanol-Gehalten im Bereich von 12,67 bis 14,16 Vol.-% erzeugt, was umgerechnet 100,0 bis 111,8 g/L entspricht. Die Glycerin-Gehalte des Weines, der mit den Hefen erhalten wird, liegen im Bereich von 9,5-16,0 g/L.

In EP 2 634 247 A1 wird eine Hefe beschrieben, die durch Fermentation von Traubenmost einen Wein erzeugt, der im niedrigsten Fall einen Ethanol-Gehalt von 15,26 Vol.-% aufweist, was umgerechnet 120,5 g/L entspricht, und einen Glycerin-Gehalt von 10,42 g/L.

Vor diesem Hintergrund bestand die Aufgabe der Erfindung darin, Hefen bereitzustellen, die bei einem gegebenen Zuckergehalt einen geringeren Ethanolgehalt und einen höheren Glyceringehalt bei der ethanolischen Fermentation erzeugen als ein eingangs verwendeter Stamm und die gleichzeitig schnell erhältlich sind.

Diese Aufgabe wird gelöst durch ein Verfahren der eingangs genannten Art, wobei das erste und das zweite Mutagen voneinander verschieden sind und aus der folgenden Gruppe ausgewählt sind: Nukleotid-alkylierendes Agens, Nukleotid-desaminierendes Agens und UV-Strahlung, wobei das erste Mutagen ein Nukleotid-alkylierendes Agens oder ein Nukleotid-desaminierendes Agens ist, und wobei zwischen dem ersten und dem zweiten Mutageneseschritt ein erster Selektionsschritt ausgeführt wird und nach dem zweiten Mutageneseschritt ein zweiter Selektionsschritt ausgeführt wird, bei denen die aus dem jeweils vorangegangenen Mutageneseschritt hervorgehenden Mutanten einem Selektionsfaktor ausgesetzt werden, der aus den folgenden Gruppen ausgewählt ist: (a) hypertones Medium und (b) Alkoholdehydrogenase-Inhibitor, wobei einer der Selektionsfaktoren aus der Gruppe (a) und einer der Selektionsfaktoren aus der Gruppe (b) ausgewählt ist.

Es hat sich gezeigt, dass bei einem derartigen Verfahren, bei dem zwei Mutageneseschritte durchgeführt werden, wobei nach jedem Mutageneseschritt zusätzlich ein Selektionsschritt ausgeführt wird, Hefen erzeugt werden, die einerseits bei einer gegebenen Zuckerkonzentration einen geringeren Ethanolgehalt bei alkoholischer Gärung bereitstellen als der verwendete Ausgangsstamm und andererseits unter denselben Bedingungen eine höhere Glycerinkonzentration erzeugen. Beides wirkt sich positiv auf den Geschmack eines mit einer solchen Hefe erzeugten Weines aus.

Der Begriff "Hefe" wie er hier verwendet wird, bezieht sich vorzugsweise auf Hefen der Gattung, *Saccharomyces,* besonders bevorzugt auf die Arten *Saccharomyces cerevisiae* oder *Saccharomyces bayanus.* Dies schließt auch Subspezies wie *Saccharomyces cerevisiae* subsp. *bayanus* mit ein.

Vorzugsweise werden bei dem erfindungsgemäßen Verfahren diploide Hefe-Stämme als eingangs verwendeter Stamm für den ersten Mutageneseschritt eingesetzt. Die Diploidie geht bei den durchgeführten Mutationen nicht verloren, so dass auch die schließlich erzeugte Hefe-Mutante diploid ist. Ein Vorteil diploider Hefen ist, dass sie Ihre Eigenschaften vergleichsweise stabil über Generationen hinweg beibehalten und daher besonders als Reinzuchthefen geeignet sind. Ebendiese Eigenschaft führt jedoch dazu, dass eine unspezifische Mutation, die beispielsweise durch Strahlung oder mutagene Agenzien ausgelöst wird, als nicht sinnvoll erschien. Es hat sich nun überraschend gezeigt, dass sich eben gerade durch die Kombination derartiger Mittel für eine unspezifische Mutation, nämlich Nukleotid-alkylierende Agenzien, Nukleotid-desaminierende Agenzien oder UV-Strahlung zusammen mit einer Selektion und erneuter Mutation und weiterer Selektion gezielt Hefe-Mutanten mit gewünschten Eigenschaften, nämlich einer verringerten Ethanol- und erhöhten Glycerinproduktion, erzeugen lassen.

Der Begriff "Nukleotid-alkylierendes Agens" beschreibt eine Substanz, die Alkylgruppen mit Basen der DNA kovalent verknüpft. Derart modifizierte Basen können zu Basenfehlpaarungen und damit zu Punktmutationen führen. Es sind zahlreiche Substanzen bekannt, die eine derartige Alkylierung bewirken.

Der Begriff "Nukleotid-desaminierendes Agens" beschreibt eine Substanz, die Aminogruppen aus Basen der DNA abspaltet. Auch durch derartige Abspaltungen werden Basenfehlpaarungen verursacht, d.h. Punktmutationen. Es sind zahlreiche Substanzen bekannt, die eine derartige Desaminierung bewirken.

Der Begriff "UV-Strahlung", wie er hierin verwendet wird, bezieht sich auf elektromagnetische Strahlung im Wellenlängenbereich von 400 nm bis 100 nm und insbesondere auf UV-C-Strahlung in einem Wellenlängenbereich von 290 nm bis 100 nm. Bevorzugt ist ein Wellenlängenbereich von 280 nm bis 240 nm, insbesondere 254 nm. Eine bevorzugte Strahlungsintensität beträgt zwischen 1000 µJ/cm² und 3000 µJ/cm², besonders bevorzugt 2000 µJ/cm². Die Einwirkung derartiger Strahlung auf DNA bewirkt die Bildung von Pyrimidindimeren, insbesondere Thymindimeren. Diese Dimere haben einen Einfluss auf die dreidimensionale Struktur der DNA und blockieren die DNA-Polymerase bei der Replikation.

Der Begriff "hypertones Medium", wie er hierin verwendet wird, bezieht sich auf ein Medium, das eine osmotisch aktive Substanz, einschließlich eines Salzes, Zuckers oder Zuckeralkohols, in einer Konzentration enthält, die eine Osmolarität von mehr als 308 mOsmol/l verursacht. Das Medium liegt flüssig oder in einer durch Zugabe von Agar verfestigten Form vor. Geeignete osmotisch aktive Substanzen sind dem Fachmann bekannt.

Ein "Alkoholdehydrogenase-Inhibitor" ist eine Substanz, die in der Lage ist, das Enzym Alkoholdehydrogenase zu inhibieren, das heißt, dessen Aktivität zu blockieren, so dass durch das Enzym, das in Hefe die Umsetzung von Acetaldehyd in Ethanol katalysiert, kein Acetaldehyd mehr umgesetzt wird. Der Inhibitor wird dabei selbst nicht umgesetzt. Es sind dem Fachmann zahlreiche Substanzen bekannt, die diese Eigenschaft aufweisen.

Bei einer Ausführungsform wird ein abschließender Testschritt durchgeführt, bei dem die in dem Verfahren erhaltenen Hefe-Mutanten darauf untersucht und ausgewählt werden, ob sie mehr Glycerin und weniger Ethanol bei einer ethanolischen Gärung erzeugen als der eingangs verwendete Hefestamm unter den gleichen Bedingungen.

Es versteht sich, dass die jeweils gleichen Bedingungen bedeuten, dass die Hefe-Mutanten und der eingangs verwendete Hefe-Stamm in dem gleichen Medium, der gleichen Atmosphäre und derselben Temperatur, möglichst zeitgleich inkubiert werden und anschließend die Konzentration an Glycerin und Ethanol ermittelt wird. Dem Fachmann sind geeignete Verfahren zur Ermittlung der Glycerinkonzentration bekannt. Für den Testschritt eingesetzte Medien sind vorzugsweise ausgewählt aus Traubenmost, der aus Weintrauben erhalten wurde, und einem künstlichen Most-Medium, das die Bedingungen eines Traubenmostes imitiert. Verschiedene derartige künstliche Most-Medien sind dem Fachmann bekannt.

In einer Ausführungsform ist das Nukleotid-alkylierendes Agens unter den folgenden ausgewählt: Dimethylsulfat (DMS), Ethylmethansulfonat (EMS), Methylmethansulfonat (MMS), 1-Methyl-3-nitro-1-nitrosoguanidin (MNNG), Methylnitrosocyanamid (MNC), Methylnitrosoharnstoff (MNU) und DNA-Methyltransferasen. Von all diesen Substanzen ist bekannt, dass sie Basen der DNA alkylieren, und es hat sich gezeigt, dass diese insbesondere für die Mutagenese bei *Saccharomyces cerevisiae* und *Saccharomyces bayanus* geeignet sind. Vorzugsweise wird für das erfindungsgemäße Verfahren Ethylmethansulfonat (EMS) eingesetzt.

In einer Ausführungsform ist das Nukleotid-desaminierendes Agens ausgewählt unter den folgenden: anorganisches Nitritsalz, organisches Nitritsalz und salpetrige Säure. Es hat sich gezeigt, dass mit diesen Nukleotid-desaminierendes Agenzien, eine für die Veränderung von *Saccharomyces cerevisiae* und *Saccharomyces bayanus* ausreichende mutagene Bedingung geschaffen wird, die andererseits jedoch das Überleben derselben ermöglicht. Vorzugsweise wird für das erfindungsgemäße Verfahren ein Natriumnitrit eingesetzt.

Bei der vorliegenden Erfindung ist das erste Mutagen ein Nukleotid-alkylierendes Agens oder ein Nukleotid-desaminierendes Agens. Es hat sich gezeigt, dass unter diesen Bedingungen eine große Anzahl an Hefe-Mutanten nach dem ersten Mutageneseschritt und Selektionsschritt erhalten werden können, die zur weiteren Mutagenese einsetzbar sind.

In einer Ausführungsform ist das erste Mutagen ein Nukleotid-alkylierendes Agens und das zweite Mutagen ein Nukleotid-desaminierendes Agens oder UV-Strahlung. Durch die spezielle Kombination eines Nukleotid-akylierenden Agens als erstem Mutagen konnten besonders viele Hefe-Mutanten erhalten werden, die in einem zweiten Mutageneseschritt einsetzbar sind. Vorzugsweise ist das zweite Mutagen ein Nukleotid-desaminierendes Agens.

In einer Ausführungsform wird das hypertone Medium durch Zugabe einer der folgenden Substanzen erhalten: Chloride und Sulfate von Natrium, Kalium, Magnesium, Calcium sowie Zucker, einschließlich Fructose und Glucose, und Zuckeralkohole, einschließlich Sorbit und Mannit. Es ist dem Fachmann bekannt, dass sich im Zusammenhang mit Hefe der Begriff hyperton darauf bezieht, dass die hypertone wässrige Lösung einen höheren osmotischen Druck als das Zytoplasma der Hefe aufweist, d.h. dass sie eine Osmolarität von mehr als 308 mOsmol/l aufweist. Bevorzugt ist eine Osmolarität im Bereich von 500 bis 700 mOsmol/l.

In einer Ausführungsform ist der Alkoholdehydrogenase-Inhibitor unter den folgenden ausgewählt; Pyrazol, 3-Methylpyrazol, 4-Methylpyrazol und Acetylsalicylsäure. Es hat sich gezeigt, dass insbesondere diese Alkohol-Dehydrogenase-Inhibitoren für die Selektion von Hefe, einschließlich Sac*charomyces cerevisiae* und *Saccharomyces bayanus,* bei der eine reduzierte Ethanolproduktion erwünscht ist, geeignet sind. Besonders bevorzugt ist die Verwendung von Pyrazol.

Bei der vorliegenden Erfindung ist einer der Selektionsfaktoren aus der Gruppe (a) und einer der Selektionsfaktoren aus der Gruppe (b) ausgewählt. Durch die Kombination zweier unterschiedlicher Selektionsfaktoren können bei dem erfindungsgemäßen Verfahren mehr Mutanten erzeugt werden, die bessere Eigenschaften hinsichtlich einer verringerten Ethanolproduktion und erhöhten Glycerinproduktion aufweisen.

In einer Ausführungsform ist der erste Selektionsfaktor aus der Gruppe (a) und der zweite Selektionsfaktor aus der Gruppe (b) ausgewählt.

In einer anderen Ausführungsform ist der erste Selektionsfaktor aus der Gruppe (b) und der zweite Selektionsfaktor aus der Gruppe (a) ausgewählt.

In einer Ausführungsform wird nach dem ersten Selektionsschritt ein Testschritt durchgeführt, bei dem nach dem ersten Selektionsschritt erhaltene intermediäre Hefe-Mutanten darauf untersucht werden, ob sie mehr Glycerin bei einer ethanolischen Gärung erzeugen als der eingangs verwendete Hefe-Stamm unter den jeweils gleichen Bedingungen, wobei nur solche intermediäre Hefe-Mutanten dem zweiten Mutageneseschritt und dem zweiten Selektionsschritt unterworfen werden, auf die dies zutrifft.

Vorzugsweise wird zusätzlich untersucht, ob die intermediären Mutanten, die mehr Glycerin erzeugen, auch weniger Ethanol bei einer ethanolischen Gärung erzeugen als der eingangs verwendete Hefe-Stamm, wobei nur solche intermediäre Hefe-Mutanten dem zweiten Mutageneseschritt und dem zweiten Selektionsschritt unterworfen werden, die mehr Glycerin und weniger Ethanol erzeugen.

Es versteht sich, dass die jeweils gleichen Bedingungen bedeuten, dass die intermediären Hefe-Mutanten und der eingangs verwendete Hefe-Stamm in dem gleichen Medium, der gleichen Atmosphäre und derselben Temperatur, möglichst zeitgleich inkubiert werden und anschließend die Konzentration an Glycerin und vorzugsweise auch Ethanol ermittelt wird. Dem Fachmann sind geeignete Verfahren zur Ermittlung der Glycerinkonzentration bzw. zur Ermittlung der Ethanolkonzentration bekannt.

Die eingangs genannte Aufgabe wird auch gelöst durch eine Hefe-Mutante, die nach einem oben beschriebenen Verfahren erhalten wurde und beim Leibnitz-Institut DSMZ-Deutsche Sammlung von Mikroorganismen und Zellkulturen GmbH unter der Zugangsnummer DSM 29822 hinterlegt ist. Es handelt sich um einen *Saccharomyces cerevisiae* subsp. *bayanus*, taxonomische Bezeichnung: *Saccharomyces cerevisiae.* Das vom Anmelder verwendete Bezugszeichen ist NP12B8 oder hierin auch Nitrit Pyra 12 B8 bzw. NO2Pyra12B8.

Der Stamm wird in einem Medium mit 10 g Hefeextrakt, 10 g Bacto Pepton, 5 g NaCl, das auf 1 I mit H2O aufgefüllt wird und dessen pH-Wert auf 7 eingestellt wird, vermehrt. Das Medium wird dazu 20 Minuten bei 121 °C sterilisiert und der pH-Wert nach dem Sterilisieren beträgt zwischen 6 und 7. Die Vermehrung erfolgt aerob bei einer Temperatur von 30 °C. Die Bebrütung erfolgt für 24 Stunden.

Diese Hefe-Mutante erzeugt beim Vergären von Most aus Rieslingtrauben, der ein durch Anreicherung erhaltenes Mostgewicht von 90° Oe (21,6 % Brix) aufweist, mit einem NOPA-Wert von 107 mg/ml (+/- 5mg/l), bei einer Dosierung der Hefe von 4 x 10⁶/ml nach 35 Tagen bei einer Gärungstemperatur von 15-25° C einen Wein, mit folgenden Anteilen an Ethanol, Glucose, Fructose, Bernsteinsäure und Glycerin:

| | |
|---|---|
| Ethanol: | 90-106 g/l |
| Glucose: | 0,18-0,42 g/l |
| Fructose: | 0,95-5,55 g/l |
| Bernsteinsäure: | 2-5 g/l |
| Glycerin: | 10-16 g/l |

Weiter zeichnet sich diese Hefemutante dadurch aus, dass sie in einem in DE 10 2006 022 569 beschriebenen Nachweisverfahren, das dort allgemein für Mikroorganismen beschrieben wird, unter Verwendung der dort beschriebenen Primer A-Not, C-Not, G-Not, T-Not in einer ersten PCR-Reaktion und Verwendung der dort beschriebenen Primer T-Not-A, T-Not-T, T-Not-G in einer zweiten PCR-Reaktion und anschließender Gelelektrophorese, die ebenfalls in DE 10 2006 022 569 beschrieben ist, das in Figur 4 dargestellte charakteristische DNA-Profil aufweist.

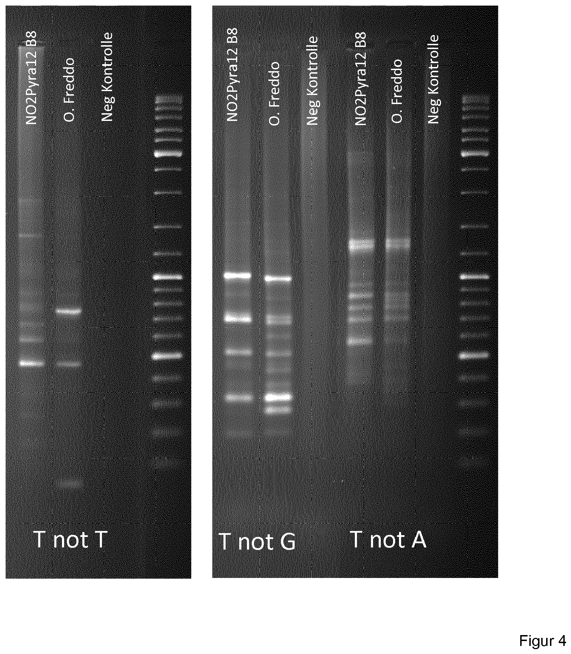

Dabei bezeichnet in Figur 4 "NO2Pyra12B8" die beim Leibnitz-Institut DSMZ-Deutsche Sammlung von Mikroorganismen und Zellkulturen GmbH unter der Zugangsnummer DSM 29822 hinterlegte Hefe, "O. Freddo" den *Saccharomyces cerevisiae* subsp. *bayanus*-Stamm LW 317-30, der unter der Bezeichnung "Oenoferm Freddo F3" im Handel weltweit erhältlich ist, und "Neg Kontrolle" eine Probe, in der keine DNA enthalten war. Als Längenstandard wurde GeneRuler DNA Ladder Mix von Thermo scientific (Fermentas) eingesetzt.

Das eingangs beschriebene Problem wird auch gelöst durch eine Hefe-Mutante, erhalten nach einem oben beschriebenen Verfahren, wobei die Mutante beim Vergären von Most mit 90° Oe (21,6 % Brix), und einem NOPA-Wert = 107 mg/l (+/- 5mg/l) bei einer Dosierung von 4x 10⁶/ml nach 35 Tagen bei einer Gärungstemperatur von 15-25 °C einen Wein mit folgenden Anteilen an Ethanol und Glycerin erzeugt:

| | |
|---|---|
| Ethanol | 70 - 150 g/l |
| Glycerin | 10 - 20 g/l |

Weiter wird das eingangs genannte Problem gelöst durch die Verwendung einer nach dem oben beschriebenen Verfahren erhaltenen Hefe-Mutante oder der beim Leibnitz-Institut DSMZ-Deutsche Sammlung von Mikroorganismen und Zellkulturen GmbH hinterlegten Hefe-Mutante mit der Zugangsnummer DSM 29822 oder einer Hefe-Mutante, die nach einem oben beschriebenen Verfahren erhalten wurde, wobei die Mutante beim Vergären von Most mit 90° Oe (21,6 % Brix), und einem NOPA-Wert = 107 mg/l (+/- 5mg/l) bei einer Dosierung von 4x 10⁶/ml nach 35 Tagen bei einer Gärungstemperatur von 15-25 °C einen Wein mit folgenden Anteilen an Ethanol und Glycerin erzeugt:

| | |
|---|---|
| Ethanol | 70 - 150 g/l, |
| Glycerin | 10 - 20 g/l, |

in einem Verfahren zur Herstellung eines alkoholhaltigen Getränks.

In einer Ausführungsform erfolgt die Verwendung so, dass das alkoholhaltige Getränk aus Traubenmost hergestellt wird. Es versteht sich, dass dabei sowohl die Verwendung zur Herstellung eines alkoholhaltigen Getränks aus Most von weißen als auch roten Trauben umfasst ist, einschließlich durch Saftabzug von roten Trauben erhaltener weißer Most (Blanc de noir) als auch Rose-Varianten davon.

Weitere Vorteile, Merkmale und Anwendungsmöglichkeiten der vorliegenden Erfindung werden deutlich anhand der folgenden Beschreibung bevorzugter Ausführungsformen und Beispiele.

Es zeigen:
- Figur 1:: Schematische Darstellung von Mutagenese- und Selektionsschritten
- Figur 2:: Gärverläufe nach einem ersten Mutagenese- und ersten Selektionsschritt
- Figur 3:: Gärverläufe nach einem zweiten Mutagenese- und zweiten Selektionsschritt
- Figur 4 :: DNA-Profile der unter der Zugangsnummer DSM29822 hinterlegten Hefe-Mutante

### Beispiele

Für die Erzeugung von Hefe-Mutanten, die eine geringe Ethanolkonzentration und erhöhte Glycerinkonzentration in einem Wein bzw. Medium bei einer gegebenen Ausgangs-Glucosekonzentration erzeugen, wurden unterschiedliche Mutationsversuche durchgeführt.

### Vorversuche

Bei zunächst durchgeführten Versuchen, unter Verwendung von Ethidiumbromid, das Mutationen durch Leserasterverschiebungen erzeugt, konnte gezeigt werden, dass dabei nur wenige lebensfähige Hefen erzeugt werden konnten, die insbesondere hinsichtlich ihrer Atmungskette beeinträchtigt sind und allgemein Veränderungen der mitochondrialen DNA aufzuweisen scheinen. Derartige Hefen sind für eine Fermentation nicht geeignet.

Weiter konnte gezeigt werden, dass eine wiederholte Anwendung desselben Mutagens dazu führt, dass nur wenige bzw. gar keine Hefe-Mutanten nach dem zweiten Mutageneseschritt und anschließender Selektion erhalten wurden.

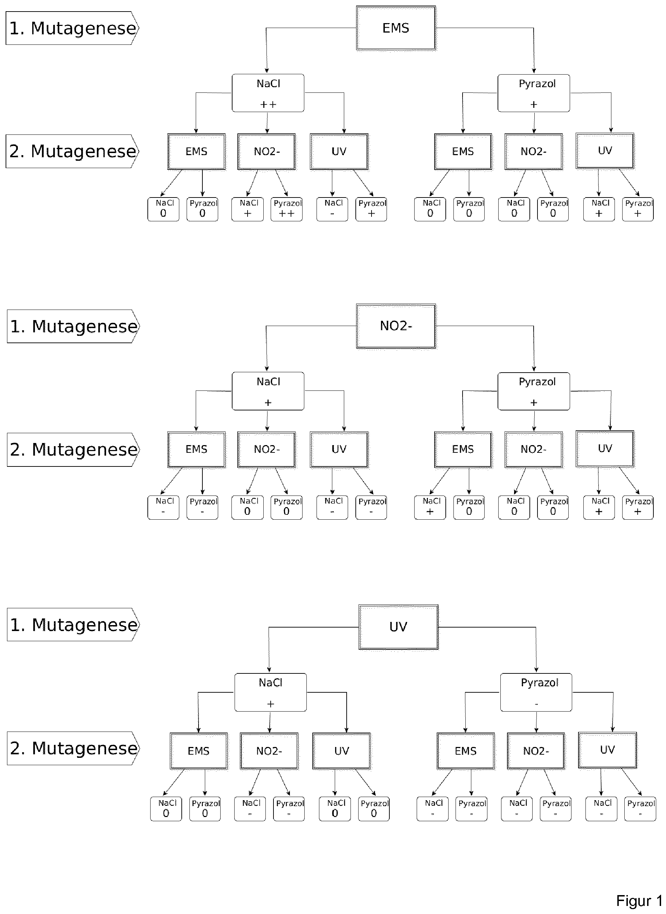

Es wurden die in Figur 1 gezeigten Mutagenese-Schemen durchgeführt, wobei "++" bedeutet, dass mehr als 100 Hefemutanten mit gewünschten Eigenschaften erhalten wurden, "+" bedeutet, dass zwischen 1 und 99 Hefemutanten mit gewünschten Eigenschaften erhalten wurden, "-" bedeutet, dass keine Hefemutanten mit gewünschten Eigenschaften erhalten wurden, und "0" bedeutet, dass überhaupt keine Hefemutanten erhalten wurden. Unter gewünschten Eigenschaften wird hier verstanden, dass die Mutanten mehr Glycerin und weniger Ethanol bei einer ethanolischen Gärung erzeugen als der eingangs verwendete Hefestamm unter den gleichen Bedingungen.

In Testgärungen konnte ermittelt werden, dass die Produktion von Glycerin allgemein unmittelbar nach Start der Fermentation erfolgt und üblicherweise bis zu 10 Tage andauert. Der Hauptteil des Glycerins wird durch Fermentation der ersten 100 g Zucker pro Liter Medium erzeugt, danach verlangsamt sich die Glycerinproduktion, wird aber grundsätzlich nicht vollständig beendet.

Im Gegensatz dazu hat sich gezeigt, dass die ethanolische Gärung, bei der Ethanol erzeugt wird, bis zu drei Wochen andauert, sodass die ethanolische Gärung und Glycerinproduktion während der Fermentation leicht überlappen.

Im Nachfolgenden werden die unterschiedlichen Schritte der Mutagenese bzw. Selektion sowie Testverfahren im Einzelnen erläutert.

### Allgemeine Übersicht

Die Mutagenese- und Selektionsschritte wurden so ausgewählt, dass sie an das Herstellungsverfahren für Wein angelehnt sind.

Nach der ersten Mutagenese und ersten Selektion wurden 10 000 Mutanten ausgewählt und auf ihre Glycerinproduktion untersucht. Unter den so überprüften 10 000 Mutanten wurden 400 ausgewählt, die die höchste Glycerinkonzentration aufweisen, und erneut auf ihre Glycerinproduktion untersucht.

Mutanten, die eine reproduzierbare erhöhte Glycerinproduktion aufwiesen, wurden einer Weinfermentation in kleinem Maßstab unterzogen und anschließend organoleptisch analysiert. Zudem erfolgte eine Ethanolbestimmung.

Die Mutante mit dem besten organoleptischen Profil, der höchsten Glycerinproduktion, der geringsten Ethanolkonzentration und der besten Fermentationskapazität wurde für den zweiten Mutageneseschritt ausgewählt. Dabei wurde das organoleptische Profil subjektiv durch Verkosten und Bewertung von oxidativem Ton, Säureeindruck, Bittergeschmack und Gesamteindruck ermittelt. Die Fermentationskapazität ergibt sich aus der Gärgeschwindigkeit, gemessen durch einen Gewichtsverlust bei der Gärung und der Dauer der Gärung, bis der vorhandene Zucker, insbesondere Glukose, verbraucht ist.

Eine Mutante wurde dann als geeignet angesehen, wenn Sie mindestens 70 % des vorhandenen Zuckers nach 35 Tagen umgesetzt hatte.

### Mutageneseschritt mit einem Nukleotid-alkylierenden Agens

Als Nukleotid-alkylierendes Agens wurde Ethylmethansulfonat (EMS) eingesetzt. Eine Kolonie eines Hefestammes wurde von Agarplatten in 5 ml YPD-Medium inokuliert und bei 28° C über Nacht angezogen. Die Zellen wurden dann durch Zentrifugation pelletiert und zweimal mit 100 mM Kaliumphosphatpuffer bei einem pH-Wert von 7 gewaschen. Danach wurden die Zellen in 10 ml eines 100 mM Kaliumphosphatpuffers bei pH-Wert 7 resuspendiert. Zu 500 µl der Zellsuspension wurden 37,5 µl reines EMS zugegeben. Die Suspension wurde eine Stunde bei 30° C auf einem Schüttler inkubiert. Die Reaktion wurde durch Zugabe von 1 ml 5%igem Natriumthiosulfat (Gew.-%) gestoppt. Die Hefezellen wurden dann einmal mit einer 5%igen wässrigen Lösung von Natriumthiosulfat gewaschen. Nach Zentrifugation wurde das Pellet in 500 µl YPD-Medium aufgenommen.

### Mutageneseschritt mit einem Nukleotid-desaminierendes Agens

Als Nukleotid-desaminierendes Agens wurde Natriumnitrit eingesetzt. Kolonien von Hefestämmen wurden von Agarplatten in 5 ml YPD-Medium inokuliert und bei 28° C über Nacht angezogen. Die Zellen wurden durch Zentrifugation pelletiert und zweimal mit 2 ml Wasser gewaschen. Nach dem Waschen wurden die Zellen in einem Gemisch aus 2 ml Wasser, 2 ml eines 0,6 M Natriumacetatpuffers mit einem pH von 4,5 und 2 ml einer 55 ml Natriumnitritlösung resuspendiert. Die Zellsuspension wurde 8 Minuten bei 30° C auf einem Schüttler inkubiert. Nach der Inkubation wurde 1 ml der Suspension mit 9 ml eines 0,67 M Kaliumphosphatpuffers mit einem pH-Wert von 7 zum Abstoppen der Reaktion gemischt.

### Mutageneseschritt mit UV-Strahlung

Kolonien von Hefestämmen wurden von Agarplatten in 5 ml YPD-Medium, das 0,3 M Natriumchlorid enthielt, inokuliert und bei 28° C über Nacht angezogen. Die Zellen wurden durch Zentrifugation pelletiert und in 10 ml KP-Medium ohne Glucose und Fructose einmal gewaschen und resuspendiert. Die optische Dichte (OD) wurde bei 600 nm gemessen und die Suspension wurde auf eine optische Dichte von 0,025 verdünnt und in eine Petrischale verbracht. Die Zellsuspension in der Petrischale wurde dann mit einer UV-Strahlung von 254 nm Wellenlänge mit einer Intensität von 2000 µJ/cm² während 45 Sekunden in einem Hoefer UVC 500 Crosslinker bestrahlt.

### Selektionsschritt mit Alkoholdehydrogenase-Inhibitor

Für die Selektion mit einem Alkoholdehydrogenase-Inhibitor wurde Pyrazol verwendet.

Für diesen Selektionsschritt wurden die aus einem Mutationsschritt erhaltenen Hefezellen auf Platten mit KP-Medium, das zusätzlich 5 g/l Pyrazol enthielt, plattiert, wobei ca. 2000 bis 3000 Zellen auf eine Platte mit den Maßen 30 x 30 cm ausgebracht wurden. Die Platten wurden 10 Tage bei 18 °C unter mikroaerophilen Bedingungen inkubiert. Mikroaerophile Bedingungen bezeichnen dabei, dass das die Platten umgebende Gasgemisch (Atmosphäre) lediglich 2 bis 10 Vol.-% Sauerstoff anstelle der für Luft sonst üblichen ca. 20 Vol.-% Sauerstoff aufwies.

### Selektionsschritt mit hypertonem Medium

Für die Verursachung von osmotischem Stress wurde bei diesem Selektionsschritt Natriumchlorid verwendet. Die aus einem Mutageneseschritt erhaltenen Hefezellen wurden dazu auf Platten mit KP-Medium, das zusätzlich 17,53 g/l Natriumchlorid enthielt, plattiert, wobei ca. 2000 bis 3000 Zellen auf eine Platte mit Maßen 30 x 30 cm ausgebracht wurden. Es wird generell angenommen, dass Mutanten, die einen Wachstumsvorteil auf hypertonen Medien haben, mehr Glycerin produzieren. Die Platten wurden 10 Tage bei 18 °C unter mikroaerophilen Bedingungen inkubiert. Mikroaerophile Bedingungen bezeichnen dabei, dass das die Platten umgebende Gasgemisch (Atmosphäre) lediglich 2 bis 10 Vol.-% Sauerstoff anstelle der für Luft sonst üblichen ca. 20 Vol.-% Sauerstoff aufwies.

### Medien

Das verwendete KP-Medium, ein Medium, das auch als künstlicher Most bezeichnet wird, das unter anderem für die Selektionsschritte verwendet wurde, enthielt pro Liter 115,5 g Glucosemonohydrat, 105 g Fructose, 3 g Weinsäure, 0,3 g Zitronensäure, 0,3 g Äpfelsäure, 0,3 g (NH₄)₂SO₄, 2 g KH₂PO₄, 0,2 g MgSO₄ x 7H₂O, 4 mg MnSO₄ x H₂O, 4 mg ZnSO₄ x 7 H₂O, 0,5 mg CuSO₄ x 5 H₂O, 0,5 mg Kl, 0,2 mg CoCl₂ x 6 H₂O, 0,5 mg (NH₄)₆Mo₇O₂₄, 0,5 mg H₃BO₃, 300 mg Myoinositol, 1 mg Nicotinsäure, 1 mg Calciumpantothenat, 1 mg Pyridoxinhydrochlorid, 0,04 mg Biotin, 1 mg p-Aminobenzoesäure, 247 mg L-Glutamin, 183 mg L-Arginin, 87,7 mg L-Tryptophan, 71 mg L-Alanin, 58,9 mg L-Glutaminsäure, 38,4 mg L-Serin, 37,1 mg L-Threonin, 23,7 mg L-Leucin, 21,8 mg L-Asparaginsäure, 21,8 mg L-Valin, 18,6 mg L-Phenylalanin, 16 mg L-Isoleucin, 16 mg L-Histidin, 15,4 mg L-Methionin, 9 mg L-Tyrosin, 9 mg L-Glycin, 8,3 mg L-Lysin und 6,4 mg L-Cystein.

Das YPD-Medium enthielt pro Liter 10 g Hefeextrakt, 20 g Pepton, 20 g Glucose und wurde auf einen pH-Wert von 5,5 bis 6,0 eingestellt.

Für Platten mit den beschriebenen Medien wurden 15 g/l Agar zu den Medien gegeben.

### Untersuchung der Glvcerinproduktion

Für die Bestimmung der Glycerinproduktion wurden Hefekolonien von einer Agarplatte in YPD-Medium inokuliert. Nach drei Tagen Wachstum bei 30° C wurde 1 ml KP-Medium mit 20 µl der so angewachsenen Kultur inokuliert. Es erfolgte keine Anpassung der Zelldichte. Die KP-Kulturen wurden dann bei 18° C unter mikroaerophilen Bedingungen inkubiert. Nach zehn Tagen wurde der Überstand der Kultur durch Zentrifugation und Filtration abgenommen. Der Überstand wurde dann hinsichtlich der Glycerinkonzentration analysiert. Die Bestimmung der Glycerinkonzentration erfolgte photometrisch. Für die Messung wurde ein Kit der R-Biopharm AG verwendet, wobei der Test für die Messung in einer Mikrotiterplatte auf ein Gesamtprobenvolumen von 155 µl angepasst wurde.

### Fermentationsversuche

Für die Fermentationsversuche wurde kein künstlicher, sondern ein echter Most eingesetzt, wobei ein Riesling des Jahrgangs 2013 mit 70° Oe (17,1 % Brix), der durch Zugabe von Saccharose (52 g) auf 90° Oe (21,6 % Brix) angereichert wurde und einen NOPA-Wert von 107 mg/l (+/- 5 mg/l) aufwies, eingesetzt wurde.

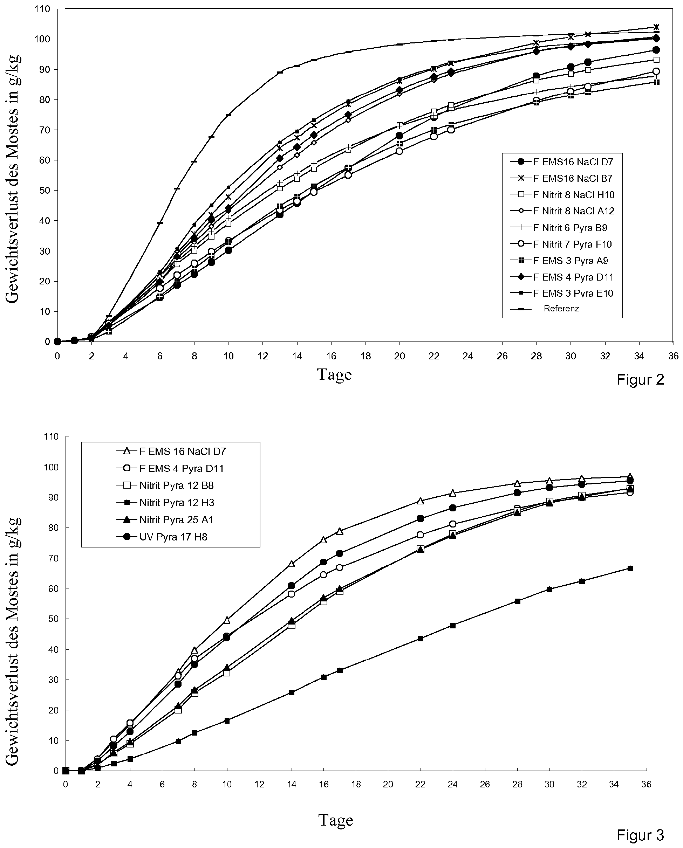

Für die Fermentation, deren Ergebnisse in Figur 2 dargestellt sind, wurde ein Most eines Rieslings des Jahrgangs 2013 eingesetzt, der auf 91 °Oe (21,7 % Brix) angereicht war und einen NOPA-Wert von 124 mg/l (+/- 5 mg/l) aufwies.

Der pH-Wert der Medien betrug zwischen 3,1 und 3,2. Die Fermentationstemperatur betrug in allen Fällen 18° C. Die Fermentationsansätze wurden nicht gerührt.

### Bestimmung von erzeugtem Glycerin, Ethanol, Glucose und Fructose sowie Gesamtzucker / organoleptische Analyse / Fermentationskapazität

Die Analyse der Testfermentationen erfolgte durch HPLC mit den folgenden Parametern:
Gerät: Ultimate 3000 ThermoScientific
Säule REZEX ROA Organic Acid H+ Phenomenex
RI Detektor & UV Detektor bei 210 nm
Laufmittel: 0,005 N H₂SO₄ (isokratisch)
Temperatur: 75 °C
Flußrate: 0,5 ml/min

Zusätzlich wurde das organoleptische Profil, das sich ebenfalls aus der Zusammensetzung des erhaltenen Weines ergibt, subjektiv durch Verkosten und Bewertung des oxidativem Tons, Säureeindrucks, Bittergeschmacks und des Gesamteindrucks ermittelt.

Die Fermentationskapazität ergibt sich aus der Gärgeschwindigkeit, gemessen durch einen Gewichtsverlust bei der Gärung und der Dauer der Gärung, bis der vorhandene Zucker, insbesondere Glukose, verbraucht ist.

### Beispiel 1:

Zur Ermittlung der unterschiedlichen Auswirkungen unterschiedlicher mutagener Reize wurden zunächst Versuche durchgeführt, bei denen ein *Saccharomyces cerevisiae* subsp. *bayanus-Stamm* UV-Strahlung, EMS oder Natriumnitrit ausgesetzt wurde. Bei dem in diesem Beispiel verwendeten Stamm handelt es sich um den unter der Bezeichnung "Oenoferm Freddo F3" weltweit erhältlichen Hefe-Stamm.

Anschließend erfolgte eine Selektion entweder durch ein hypertones Natriumchlorid-Medium oder auf einem Pyrazol-Medium.

Ergebnisse der Untersuchung einiger Stämme, die aus der Mutation mit EMS bzw. Natriumnitrit hervorgegangen sind, sind in Figur 2 dargestellt. Figur 2 zeigt dabei graphisch den Gewichtsverlust bei einer Testfermentation über insgesamt 35 Tage, wobei das Gesamtgewicht des eingesetzten Mostes durch Wiegen bestimmt wurde und die Angabe des Gewichtsverlustes sich auf das Gewicht des ursprünglich eingesetzten Mostes mit Hefe bezieht. Je größer der Gewichtsverlust, desto besser ist die fermentative Kapazität des jeweiligen Stammes.

In der unten angefügten Tabelle 1 sind die Ergebnisse der HPLC-Analyse zu dieser Testfermentation nach 35 Tagen dargestellt, wobei zu jeder Mutante jeweils zwei voneinander unabhängige Fermentationsansätze untersucht wurden.

**Tabelle1:**

| **Probe** | **Glucose g/l** | **Fructose g/l** | **Gesamtzucker g/l** | **Ethanol g/l** | **Glycerin g/l** |
|---|---|---|---|---|---|
| F EMS16 NaCl D7 | 2,53 | 14,83 | 17,36 | 100,2 | 8,97 |
| F EMS16 NaCl D7 | 0,26 | 4,28 | 4,54 | 105,0 | 9,75 |
| F EMS16 NaCl B7 | 0,29 | 4,62 | 4,91 | 105,7 | 6,57 |
| F EMS16 NaCl B7 | 0,02 | 0,23 | 0,25 | 107,1 | 6,57 |
| F Nitrit 8 NaCl H10 | 0,07 | 1,38 | 1,45 | 107,1 | 6,64 |
| F Nitrit 8 NaCl H10 | 6,66 | 26,25 | 32,91 | 92,6 | 6,24 |
| F Nitrit 8 NaCl A12 | 0,04 | 1,31 | 1,35 | 106,4 | 6,75 |
| F Nitrit 8 NaCl A12 | 0,38 | 4,82 | 5,20 | 107,1 | 6,51 |
| F Nitrit 6 Pyra B9 | 12,04 | 39,09 | 51,13 | 85,2 | 5,84 |
| F Nitrit 6 Pyra B9 | 0,03 | 0,60 | 0,63 | 111,4 | 6,88 |
| F Nitrit 7 Pyra F10 | 3,59 | 19,85 | 23,44 | 100,2 | 6,54 |
| F Nitrit 7 Pyra F10 | 3,56 | 19,31 | 22,87 | 98,1 | 6,57 |
| F EMS 3 Pyra A9 | 18,37 | 45,85 | 64,22 | 79,8 | 5,72 |
| F EMS 3 Pyra A9 | 0,09 | 2,34 | 2,43 | 109,2 | 6,93 |
| F EMS 4 Pyra D11 | 0,26 | 4,03 | 4,29 | 105,7 | 8,06 |
| F EMS 4 Pyra D11 | 0,00 | 0,31 | 0,31 | 108,5 | 8,14 |
| F EMS 3 Pyra E10 | 0,00 | 0,77 | 0,77 | 109,2 | 7,09 |
| F EMS 3 Pyra E10 | 0,00 | 2,55 | 2,55 | 110,7 | 7,01 |
| Referenz | 0,00 | 0,21 | 0,21 | 112,1 | 7,17 |
| Referenz | 0,00 | 0,19 | 0,19 | 109,9 | 7,25 |

In Figur 2 und Tabelle 1 sind Hefen, die zunächst einem Mutageneseschritt mit EMS unterzogen wurden und anschließend einer Selektion mit NaCl, mit "F EMS 16 NaCl E7" und "F EMS 16 NaCl B7" bezeichnet. Stämme, die zunächst einem Mutageneseschritt mit Nitrit und einer Selektion mit NaCl ausgesetzt wurden, sind mit "F Nitrit 8 NaCl H10" und "F Nitrit 8 NaCl A12" bezeichnet, Stämme, die zunächst einem Mutageneseschritt mit Nitrit und anschließend einer Selektion mit Pyrazol unterzogen wurden, sind mit "F Nitrit 6 Pyra B9" und "F Nitrit 7 Pyra F10" bezeichnet und Stämme, die zunächst einem Mutageneseschritt mit EMS und anschließend einer Selektion mit Pyrazol unterzogen wurden, sind mit "F EMS 3 Pyra A9", "F EMS 4 Pyra D11" und "F EMS 3 Pyra E10" bezeichnet. Die Bezeichnung "Referenz" bezieht sich auf den als Vergleichshefe eingesetzten *Saccharomyces cerevisiae* subsp. *bayanus*-Stamm, der auch eingangs für den ersten Mutageneseschritt verwendet wurde.

Es zeigt sich, dass durch diesen ersten Mutageneseschritt und anschließenden Selektionsschritt sowohl Mutanten erhalten werden, die weniger Glycerin produzieren als die Vergleichshefe unter gleichen Bedingungen als auch mehr Glycerin produzieren als die Vergleichshefe unter gleichen Bedingungen. Grundsätzlich ist dabei allerdings zu berücksichtigen, dass ein Teil der intermediären Mutanten nach 35 Tagen nicht allen Zucker umgesetzt hatte, so dass die Glycerinwerte bei diesen Versuchen nur bedingt vergleichbar sind

### Beispiel 2:

Bei diesem Beispielversuch wurde der aus Beispiel 1 erhaltene Stamm "F EMS 16 NaCl D7", der sich durch eine besonders hohe Glycerinkonzentration in der Testfermentation auszeichnete, für einen zweiten Mutageneseschritt und Selektionsschritt ausgewählt. Für den zweiten Mutageneseschritt wurden ebenfalls EMS, Natriumnitrit und UV-Strahlung eingesetzt. Eine anschließende Selektion erfolgte jeweils mit Natriumchlorid oder Pyrazol. Dabei zeigte sich, dass bei einer weiteren Mutation mit EMS bei dem zuvor bereits mit EMS mutierten Stamm keine lebensfähigen Mutanten erhalten wurden.

Beispielhaft sind in Figur 3 Ergebnisse eines Fermentationsversuches mit unterschiedlichen Mutanten, die nach dem zweiten Mutageneseschritt und der zweiten Selektion erhalten wurden, gezeigt.

Dabei bezeichnen "F EMS 16 NaCl D7" und "F EMS 4 Pyra D11" intermediäre Mutanten. Sie stellen in diesem Beispiel Vergleichswerte dar.

"Nitrit Pyra 12 B8", "Nitrit Pyra 12 H3" und "Nitrit Pyra 25 A1" bezeichnen Stämme, bei denen der zweite Mutageneseschritt mit Natriumnitrit und der zweite Selektionsschritt mit Pyrazol erfolgte. UV Pyra 17 A8 bezeichnet einen Stamm, bei dem der zweite Mutageneseschritt mit UV-Strahlung und die zweite Selektion mit Pyrazol erfolgte.

In Tabelle 2 sind die Ergebnisse der HPLC-Untersuchungen dieser Stämme dargestellt. Auch hier zeigt sich, dass teilweise Stämme erhalten wurden, die deutlich mehr Glycerin produzieren als die intermediär in Beispiel 1 erhaltenen Mutanten, und teilweise Stämme erhalten wurden, die weniger Glycerin erzeugen. Es wurden insgesamt nur wenige Mutanten erzeugt, die deutlich weniger Ethanol produzieren als die eingangs des zweiten Mutageneseschrittes eingesetzte intermediäre Mutante. Der erzeugte Stamm "Nitrit Pyra 12 H3" zeichnet sich dadurch aus, dass weder Glucose noch Fructose vollständig umgesetzt werden, was auf eine unvollständig ablaufende Fermentation hindeutet.

Die in Tabelle 2 dargestellten Daten wurden nach 35 Tagen Testfermentation erhalten, wobei für jede Mutante unabhängig voneinander zwei Fermentationsansätze durchgeführt und ausgewertet wurden.

**Tabelle 2:**

| **Probe** | **Glucose g/l** | **Fructose g/l** | **Gesamt-zucker g/l** | **Ethanol g/l** | **Glycerin g/l** |
|---|---|---|---|---|---|
| F EMS 16 NaCl D7 | 0,03 | 1,25 | 1,28 | 102,9 | 8,23 |
| F EMS 16 NaCl D7 | 0,01 | 0,24 | 0,25 | 101,6 | 8,48 |
| F EMS 4 Pyra D11 | 0,06 | 1,49 | 1,55 | 105,0 | 6,65 |
| F EMS 4 Pyra D11 | 0,95 | 8,02 | 8,97 | 100,9 | 6,38 |
| Nitrit Pyra 12 B8 | 0,18 | 3,58 | 3,76 | 98,8 | 11,31 |
| Nitrit Pyra 12 B8 | 0,42 | 5,55 | 5,97 | 96,7 | 11,08 |
| Nitrit Pyra 12 H3 | 21,02 | 51,82 | 72,84 | 85,8 | 10,55 |
| Nitrit Pyra 12 H3 | 10,90 | 34,44 | 45,34 | 77,2 | 10,86 |
| Nitrit Pyra 25 A1 | 0,70 | 4,77 | 5,47 | 101,6 | 8,73 |
| Nitrit Pyra 25 A1 | 0,76 | 4,85 | 5,61 | 100,9 | 8,74 |
| UV Pyra 17 H8 | 0,02 | 0,72 | 0,74 | 103,6 | 8,52 |
| UV Pyra 17 H8 | 0,15 | 3,20 | 3,35 | 102,2 | 8,42 |

Zusammenfassend zeigte sich bei den Versuchen, dass die Mutante "Nitrit Pyra 12 B8" nach 35 Tagen eine fast vollständige Fermentation bereitstellt und in beiden Fermentationsansätzen mehr als 11 g/l Glycerin erzeugte. Diese Mutante entspricht dem beim Leibnitz-Institut DSMZ-Deutsche Sammlung von Mikroorganismen und Zellkulturen GmbH unter der Zugangsnummer DSM 29822 hinterlegten Stamm.

## Patentansprüche

1. Verfahren zur Erzeugung von Hefe-Mutanten, bei dem man wenigstens einen Hefe-Stamm in einem ersten Mutageneseschritt mit einem ersten Mutagen in Kontakt bringt und in einem zweiten Mutageneseschritt mit einem zweiten Mutagen in Kontakt bringt, wobei das Verfahren **dadurch gekennzeichnet ist, dass**
- das erste und das zweite Mutagen voneinander verschieden sind und aus der folgenden Gruppe ausgewählt sind:
Nukleotid-alkylierendes Agens, Nukleotid-desaminierendes Agens und UV-Strahlung,
wobei das erste Mutagen ein Nukleotid-alkylierendes Agens oder ein Nukleotid-desaminierendes Agens ist,
und
- zwischen dem ersten und dem zweiten Mutageneseschritt ein erster Selektionsschritt ausgeführt wird und nach dem zweiten Mutageneseschritt ein zweiter Selektionsschritt ausgeführt wird, bei denen die aus dem jeweils vorangegangenen Mutageneseschritt hervorgehenden Mutanten einem Selektionsfaktor ausgesetzt werden, der aus den folgenden Gruppen ausgewählt ist:
(a) hypertones Medium und
(b) Alkoholdehydrogenase-Inhibitor,
wobei einer der Selektionsfaktoren aus der Gruppe (a) und einer der Selektionsfaktoren aus der Gruppe (b) ausgewählt ist.

2. Verfahren nach Anspruch 1, **dadurch gekennzeichnet, dass** das Nukleotid-alkylierende Agens unter den folgenden ausgewählt ist: Dimethylsulfat (DMS), Ethylmethansulfonat (EMS), Methylmethansulfonat (MMS), 1-Methyl-3-nitro-1-nitrosoguanidin (MNNG), Methylnitrosocyanamid (MNC), Methylnitrosoharnstoff (MNU) und DNA-Methyltransferasen.

3. Verfahren nach einem der Ansprüche 1 und 2, **dadurch gekennzeichnet, dass** das Nukleotid-desaminierende Agens unter den folgenden ausgewählt ist: anorganisches Nitritsalz, organisches Nitritsalz und salpetrige Säure.

4. Verfahren nach einem der Ansprüche 1 bis 3, **dadurch gekennzeichnet, dass** das erste Mutagen ein Nukleotid-alkylierendes Agens ist und das zweite Mutagen ein Nukleotid-desaminierendes Agens oder UV-Strahlung ist.

5. Verfahren nach einem der Ansprüche 1 bis 4, **dadurch gekennzeichnet, dass** das hypertone Medium durch Zugabe einer der folgenden Substanzen erhalten wird: Chloride und Sulfate von Natrium, Kalium, Magnesium, Calcium sowie Zucker, einschließlich Fructose und Glucose, und Zuckeralkohole, einschließlich Sorbit und Mannit.

6. Verfahren nach einem der Ansprüche 1 bis 5, **dadurch gekennzeichnet**, das der Alkoholdehydrogenase-Inhibitor unter den folgenden ausgewählt ist: Pyrazol, 3-Methylpyrazol, 4-Methylpyrazol und Acetylsalicylsäure.

7. Verfahren nach einem der Ansprüche 1 bis 6, **dadurch gekennzeichnet, dass** nach dem ersten Selektionsschritt ein Testschritt durchgeführt wird, bei dem nach dem ersten Selektionsschritt erhaltene intermediäre Hefe-Mutanten darauf untersucht werden, ob sie mehr Glycerin bei einer ethanolischen Gärung erzeugen als der eingangs verwendete Hefe-Stamm unter den jeweils gleichen Bedingungen, wobei nur solche intermediäre Hefe-Mutanten dem zweiten Mutageneseschritt und dem zweiten Selektionsschritt unterworfen werden, auf die dies zutrifft.

8. Verfahren nach einem der Ansprüche 1 bis 7, **dadurch gekennzeichnet, dass** hierdurch eine Hefe-Mutante erzeugt wird, wie sie beim Leibniz-Institut DSMZ-Deutsche Sammlung von Mikroorganismen und Zellkulturen GmbH unter der Zugangsnummer DSM 29822 hinterlegt ist.

9. Verfahren nach einem der Ansprüche 1 bis 7, **dadurch gekennzeichnet, dass** hierdurch eine Hefe-Mutante erzeugt wird, die beim Vergären von Most mit 90° Oe (21,6 % Brix), und einem NOPA-Wert = 107 mg/l bei einer Dosierung von 4x 10⁶/ml nach 35 Tagen bei einer Gärungstemperatur von 15-25 °C einen Wein mit folgenden Anteilen an Ethanol und Glycerin erzeugt:
| | |
|---|---|
| Ethanol | 70 - 150 g/l |
| Glycerin | 10 - 20 g/l. |

10. Hefe-Mutante, erhalten nach einem Verfahren nach einem der Ansprüche 1 bis 7 und hinterlegt beim Leibniz-Institut DSMZ-Deutsche Sammlung von Mikroorganismen und Zellkulturen GmbH unter der Zugangsnummer DSM 29822.

11. Verwendung der Hefe-Mutante nach Anspruch 10 in einem Verfahren zur Herstellung eines alkoholhaltigen Getränks.

12. Verwendung nach Anspruch 11, **dadurch gekennzeichnet, dass** das alkoholhaltige Getränk aus Traubenmost hergestellt wird.

## Claims

1. Method for producing yeast mutants, wherein at least one yeast strain is contacted in a first mutagenesis step with a first mutagen and in a second mutagenesis step with a second mutagen, whereby the method is **characterised by**
- the first and the second mutagen being different from each other and being selected from the following group:
nucleotide-alkylating agent, nucleotide-deaminating agent and UV radiation,
wherein the first mutagen is a nucleotide-alkylating agent or a nucleotide-deaminating agent,
and
- a first selection step being performed between the first and second mutagenesis step and a second selection step being performed after the second mutagenesis step, in which the mutants resulting from the preceding mutagenesis step are exposed to a selection factor selected from the following groups:
(a) hypertonic medium and
(b) alcohol-dehydrogenase inhibitor,
wherein one of the selection factors is selected from Group (a) and one of the selection factors from Group (b).

2. Method according to Claim 1, **characterised in that** the nucleotide-alkylating agent is selected from the following: dimethylsulphate (DMS), ethyl methanesulphonate (EMS), methyl methanesulphonate (MMS), 1-methyl-3-nitro-1-nitrosoguanidine (MNNG), methylnitrosocyanamide (MNC), methylnitrosourea (MNU) and DNA methyltransferases.

3. Method according to one of Claims 1 and 2, **characterised in that** the nucleotide-deaminating agent is selected from the following: anorganic nitrite salt, organic nitrite salt and nitrous acid.

4. Method according to one of Claims 1 to 3, **characterised in that** the first mutagen is a nucleotide-alkylating agent and the second mutagen is a nucleotide-deaminating agent or UV radiation.

5. Method according to one of Claims 1 to 4, **characterised in that** the hypertonic medium is obtained by addition of one of the following substances: chlorides and sulphates of sodium, potassium, magnesium, calcium and sugar, including fructose and glucose and sugar alcohols, including sorbitol and mannitol.

6. Method according to one of Claims 1 to 5, **characterised in that** the alcohol dehydrogenase inhibitor is selected from the following: pyrazole, 3-methylpyrazole, 4-methylpyrazole and acetyl salicylic acid.

7. Method according to one of Claims 1 to 6, **characterised in that** a test step is performed after the first selection step, in which intermediate yeast mutants obtained after the first selection step are tested for whether they produce more glycerol during an ethanolic fermentation than the initially used yeast strain under the same conditions, whereby only such intermediate yeast mutants to which this applies are subjected to the second mutagenesis step and the second selection step.

8. Method according to one of Claims 1 to 7, **characterised in that** it produces a yeast mutant, such as the one deposited at the Leibniz-Institut DSMZ-Deutsche Sammlung von Mikroorganismen und Zellkulturen GmbH under accession number DSM 29822.

9. Method according to one of Claims 1 to 7, **characterised in that** it produces a yeast mutant, which produces by fermentation of must with 90° Oe (21.6 % Brix) and a NOPA value = 107 mg/l at a dosing of 4x 10⁶/ml, after 35 days at a fermentation temperature of 15-25 °C a wine with the following proportions of ethanol and glycerol:
| | |
|---|---|
| Ethanol | 70-150 g/l |
| Glycerol | 10-20 g/l. |

10. Yeast mutant obtained by a method according to one of Claims 1 to 7 and deposited at the Leibniz-Institut DSMZ-Deutsche Sammlung von Mikroorganismen und Zellkulturen GmbH under accession number DSM 29822.

11. Use of the yeast mutant according to Claim 10 in a method for producing an alcoholic beverage.

12. Use according to Claim 11, **characterised in that** the alcoholic beverage is produced from grape must.

## Revendications

1. Procédé de production de mutants de levure, dans lequel, dans une première étape de mutagénèse, on met en contact au moins une souche de levure avec un premier mutagène, et dans une seconde étape de mutagénèse avec un second mutagène, le procédé étant **caractérisé en ce que**
- le premier et le second mutagènes sont différents l'un de l'autre et sont choisis dans le groupe suivant :
un agent d'alkylation des nucléotides, un agent de désamination des nucléotides, et un rayonnement UV,
le premier mutagène étant un agent d'alkylation des nucléotides ou un agent de désamination des nucléotides,
et **en ce que**
- entre la première et la seconde étapes de mutagénèse, on met en oeuvre une première étape de sélection et, après la seconde étape de mutagénèse, on met en oeuvre une seconde étape de sélection, les mutants obtenus dans chacune des étapes de mutagénèse ci-dessus étant exposés à un facteur de sélection qui est choisi dans les groupes suivants :
(a) un milieu hypertonique, et
(b) un inhibiteur d'alcool-déshydrogénase,
l'un des facteurs de sélection étant choisi dans le groupe (a) et l'un des facteurs de sélection étant choisi dans le groupe (b).

2. Procédé selon la revendication 1, **caractérisé en ce que** l'agent d'alkylation des nucléotides est choisi parmi les suivants : sulfate de diméthyle (DMS), méthanesulfonate d'éthyle (EMS), méthanesulfonate de méthyle (MMS), 1-méthyl-3-nitro-1-nitrosoguanidine (MNNG), méthylnitrosocyanamide (MNC), méthylnitroso-urée (MNU) et ADN-méthyltransférases.

3. Procédé selon l'une des revendications 1 et 2, **caractérisé en ce que** l'agent de désamination des nucléotides est choisi parmi les suivants : sel nitrite inorganique, sel nitrite organique et acide nitreux.

4. Procédé selon l'une des revendications 1 à 3, **caractérisé en ce que** le premier mutagène est un agent d'alkylation des nucléotides et le second mutagène est un agent de désamination des nucléotides ou un rayonnement UV.

5. Procédé selon l'une des revendications 1 à 4, **caractérisé en ce que** le milieu hypertonique est obtenu par addition de l'une des substances suivantes : des chlorures et sulfates de sodium, de potassium, de magnésium, de calcium, ainsi que des sucres, y compris le fructose et le glucose, et des sucre-alcools, y compris le sorbitol et le mannitol.

6. Procédé selon l'une des revendications 1 à 5, **caractérisé en ce que** l'inhibiteur d'alcool-déshydrogénase est choisi parmi les suivants : le pyrazole, le 3-méthylpyrazole, le 4-méthylpyrazole et l'acide acétylsalicylique.

7. Procédé selon l'une des revendications 1 à 6, **caractérisé en ce qu'**on met en oeuvre après la première étape de sélection une étape d'essai, dans laquelle on étudie les mutants de levure intermédiaires obtenus après la première étape de sélection, pour savoir s'ils produisent, lors d'une fermentation éthanolique, plus de glycérol que la souche de levure initialement utilisée, dans chaque cas dans les mêmes conditions, seuls étant soumis à la seconde étape de mutagénèse et à la seconde étape de sélection les mutants de levure intermédiaires auxquels cette règle s'applique.

8. Procédé selon l'une des revendications 1 à 7, **caractérisé en ce qu'**on produit par ce biais un mutant de levure tel que déposé auprès de l'établissement Leibniz-Institut DSMZ-Deutsche Sammlung von Mikroorganismen und Zellkulturen GmbH sous le numéro d'accession DSM 29822.

9. Procédé selon l'une des revendications 1 à 7, **caractérisé en ce qu'**on produit par ce biais un mutant de levure qui, lors de la fermentation d'un moût à 90° Oe (21,6 % Brix) et une valeur à l'essai NOPA de 107 mg/l pour un dosage de 4 x 10⁶/ml, au bout de 35 jours à une température de fermentation de 15 à 25 °C, donne un vin contenant les fractions suivantes d'éthanol et de glycérol :
| | |
|---|---|
| Éthanol | 70 - 150 g/l |
| Glycérol | 10 - 20 g/l. |

10. Mutant de levure obtenu par un procédé selon l'une des revendications 1 à 7 et déposé auprès de l'établissement Leibniz-Institut DSMZ-Deutsche Sammlung von Mikroorganismen und Zellkulturen GmbH sous le numéro d'accession DSM 29822.

11. Utilisation du mutant de levure selon la revendication 10 dans un procédé de fabrication d'une boisson alcoolisée.

12. Utilisation selon la revendication 11, **caractérisée en ce que** la boisson alcoolisée est fabriquée à partir de moût de raisin.
